# EUROPÄISCHE PATENTSCHRIFT

(11) **EP 2 865 739 B1**
(45) Veröffentlichungstag und Bekanntmachung des Hinweises auf die Patenterteilung: **19.09.2018**
(21) Anmeldenummer: 13190502.8
(22) Anmeldetag: 28.10.2013
(51) Int. Cl.: C11B 9/00, A61Q 15/00, A61L 9/01, C11D 3/00, C11D 3/20

(54) **Verwendung von Lactonen**
Use of lactones
Utilisation de lactones

(43) Veröffentlichungstag der Anmeldung: 29.04.2015
(73) Patentinhaber: Symrise AG, 37603 Holzminden (DE)
(72) Erfinder: Schulze, Nicole, 31084 Freden (DE); Reindl, Hans-Georg, 37603 Holzminden (DE); Betzer, Marcus, 37603 Holzminden (DE)
(74) Vertreter: Fabry, Bernd

(56) Entgegenhaltungen:
- EP-A1- 2 386 211
- EP-A2- 1 332 772
- WO-A1-96/04940
- WO-A1-03/070871
- WO-A1-2004/009750
- US-A1- 2002 197 287
- US-A1- 2004 242 452
- US-A1- 2006 207 037

## Beschreibung

### Gebiet der Erfindung

Die Erfindung befindet sich auf dem Gebiet der oleochemischen Produkte und betrifft die Verwendung von speziellen Lactonen zur Geruchsverbesserung.

### Stand der Technik

Oleochemische Rohstoffe, wie Fette und Öle sowie deren Derivate der ersten und zweiten Ordnung stellen wichtige Ausgangsstoffe für die Herstellung sowohl von Wasch-, Spül- und Reinigungsmitteln, aber auch von kosmetischen Produkten dar. Die Triglyceride und die daraus durch Umesterung gewonnenen Fettsäureester dienen beispielsweise als Ölkörper, aus den durch Verseifung oder Hydrierung hergestellten Derivaten werden anionische und nichtionische Tenside, wie z.B. Seifen, Fettalkoholsulfate, Fettalkoholethersulfate oder Fettalkoholpolyglykolether gewonnen. Gemeinsam ist allen diesen Stoffen, dass sie einen typischen fettigen Geruch besitzen, der bei längerer Lagerung zudem weitere unangenehme Noten entwickelt. Ursache hierfür sind neben kurzkettigen freien Fettsäuren, die durch Verseifung der Triglyceride freigesetzt werden, insbesondere Aldehyde und andere Stoffe, die durch Oxidation entstehen. In der Regel ist die geruchliche Qualität der oleochemischen Stoffe nicht ausreichend, um sie ohne weiteres in die verschiedenen Endprodukte einzusetzen.

Eine Methode, dieses Problem zu lösen, besteht darin, geruchlich nicht einwandfreie Chargen einer Nachbehandlung, der so genannten Desodorierung, zu unterwerfen. Dabei werden die Rohstoffe mit heißem Wasserdampf behandelt, wobei die Geruchsträger zu einem großen Teil abdestilliert werden. Das Verfahren ist jedoch mit hohem Energieeintrag verbunden. Insbesondere dann, wenn es darum geht, möglichst preiswerte Fette und Öle zu verarbeiten, die naturgemäß den höchsten Anteil an den genannten unerwünschten Geruchsträgern aufweisen, ist diese Maßnahme ökonomisch gesehen natürlich kontraproduktiv.

In manchen Fällen ist es auch möglich, den unangenehmen Geruch durch Zugabe einers Verkapselungsmittels einzuschließen, wie dies beispielsweise in WO 2009 131748 A1 (Belle Air) beschrieben wird. Allerdings ist auch diese Maßnahme aus ökonomischer Sicht kaum zu realisieren.

Die gängigste Alternative besteht bislang darin, den geruchlich nicht einwandfreien Rohstoffen - oder den damit konfektionierten Endprodukten - Duftstoffe beizufügen, die den unangenehmen Geruch überdecken (maskieren) oder sich mit diesem in einer Art und Weise kombinieren, dass ein neuer Geruch entsteht, der weniger unangenehm ist. Auch dieses Verfahren ist aufwendig und teuer, da bislang stets erhebliche Mengen an solchen Maskierungsstoffen erforderlich gewesen sind.

Die Aufgabe der vorliegenden Erfindung hat somit darin bestanden, neue Wirkstoffe zur Verfügung zu stellen, die schon in sehr geringen Konzentrationen in der Lage sind, die in oleochemischen Rohstoffen und in den damit hergestellten Endprodukten auftretenden unangenehmen Duftnoten, wie beispielsweise fettiger oder ranziger Geruch, entgegenzuwirken, d.h. zu überdecken oder sich mit ihnen zu verbinden, so dass ein neuer angenehmer, beispielsweise süßer, cremiger oder aromatischer Geruch entsteht.

### Beschreibung der Erfindung

Gegenstand der Erfindung ist die Verwendung einer Mischung enthaltend (a) γ-Decalacton und (b) γ-Undecalacton zur Maskierung von geruchlichen Fehlnoten in oleochemischen Zubereitungen. Überraschenderweise wurde gefunden, dass diese Lactone gerade in geringen Mengen in der Lage sind, unangenehme Gerüche in einer Vielzahl von oleochemichen Formulierungen, die insbesondere fettige oder ranzige Noten aufweisen, nicht nur zu maskieren, sondern sogar angenehme Geruchsnoten zu verstärken und in den Vordergrund zu rücken.

### Lactone

Die Alkyllactone der vorliegenden Erfindung stellen bekannte Stoffe dar, die nach den einschlägigen Verfahren der organischen Chemie erhalten werden können. Eine einfache Methode zur Herstellung der 5-gliedrigen γ-Lactone besteht darin, wässrige Lösungen entsprechender Hydroxycarbonsäuren einzudampfen. Eine andere Methode, Lactone herzustellen, stellt die Baeyer-Villinger Oxidation dar, bei der cyclische Ketone mit Peroxycarbonsäuren unter Ringerweiterung oxidiert werden.

Die Lactone dienen dabei speziell zur Maskierung von Geruchsnoten, die durch freie Fettsäuren, Aldehyde sowie Oxidationsprodukte von Fetten und Ölen in oleochemischen Produkten hervorgerufen werden.

Im Sinne der vorliegenden Erfindung lassen sich die Lactone in beliebige Produkte einsetzen, in denen es gilt, unangenehme Geruchsnoten, die vor allem durch Fettderivate hervorgerufen werden, zu maskieren bzw. durch angenehme Geruchswahrnehmungen zu ersetzen. Typische Beispiele für Produktgruppen, in denen die Lactone eingesetzt werden können, sind beispielsweise Seifenbasen, Wasch-, Spül- und Reinigungsmittel sowie kosmetische Zubereitungen. Vorzugsweise werden die Lactone in Mengen von 0,01 bis 1 Gew.-%, insbesondere 0,02 bis 0,5 Gew.-% und besonders bevorzugt 0,05 bis 0,1 Gew.-% - bezogen auf die Zubereitungen - eingesetzt.

### Wasch-, Spül- und Reinigungsmittel

Ein weiterer Gegenstand der Erfindung betrifft Wasch-, Spül- und Reinigungsmittel, enthaltend mindestens ein Lacton der Formel (I), wobei die Einsatzkonzentration des Lactons oder der Lactone üblicherweise im Bereich von 0,01 bis 1 Gew.-%, vorzugsweise 0,1 bis 0,5 Gew.-% - bezogen auf die Zubereitungen - beträgt.

Die Wasch-, Spül- und Reinigungsmittel liegen vorzugsweise flüssig vor und können weitere handelsübliche Bestandteile aufweisen, wie beispielsweise Tenside, Gerüststoffe, Bleichmittel, Bleichmittelaktivatoren, Verdickungsmittel, Enzyme, Elektrolyte, pH-Stellmittel, Farb- und Duftstoffe, Schauminhibitoren, Antiredepositionsmittel, optische Aufheller, Vergrauungsinhibitoren, Knitterschutzmittel, antimikrobielle Wirkstoffe, Konservierungsmittel, Antioxidantien, Antistatika, UV-Adsorber, Schwermetallkomplexbildner und dergleichen. Diese Hilfsstoffe werden im Folgenden näher beschrieben:

### A1. Tenside

Als Tenside zur Herstellung der Wasch- oder Reinigungsmittel können neben den nichtionischen Tensiden auch Anion-, Kation-, Ampho- und/oder Niotenside und verzweigte Alkylsulfaten verwendet werden.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C12-14-Alkohole mit 3 EO, 4 EO oder 7 EO, C9-11-Alkohol mit 7 EO, C13-5-Alkohole mit 3 EO, 5 EO, 7 EO oder 8 EO, C12-18-Alkohole mit 3 EO, 5 EO oder 7 EO und Mischungen aus diesen, wie Mischungen aus C12-4-Alkohol mit 3 EO und C12-8-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise, sondern statistisch verteilt sind. Solche Produkte sind durch gleichzeitige Einwirkung von Ethylen- und Propylenoxid auf Fettalkohole erhältlich.

Eine weitere Klasse von nichtionischen Tensiden, die vorteilhaft zur Herstellung von Wasch- oder Reinigungsmitteln eingesetzt werden kann, sind die Alkylpolyglycoside (APG). Einsetzbare Alkypolyglycoside genügen der allgemeinen Formel RO(G)Z, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosidierungsgrad z liegt dabei zwischen 1,0 und 4,0, vorzugsweise zwischen 1,0 und 2,0 und insbesondere zwischen 1,1 und 1,4.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können zur Herstellung der Wasch- oder Reinigungsmittel geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel

R-CO-N(R1)-[Z], in der RCO für einen aliphatischen Acylrest mit 6 bis 22 Kohlenstoffatomen, R1 für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Zur Gruppe der Polyhydroxyfettsäureamide gehören auch Verbindungen der Formel R-CO-N(R1-O-R2)- [Z], in der R für einen linearen oder verzweigten Alkyl- oder Alkenylrest mit 7 bis 12 Kohlenstoffatomen, R1 für einen linearen, verzweigten oder cyclischen Alkylrest oder einen Arylrest mit 2 bis 8 Kohlenstoffatomen und R2 für einen linearen, verzweigten oder cyclischen Alkylrest oder einen Arylrest oder einen Oxy-Alkylrest mit 1 bis 8 Kohlenstoffatomen steht, wobei C1-4-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes. [Z] wird vorzugsweise durch reduktive Aminierung eines Zuckers erhalten, beispielsweise Glucose, Fructose, Maltose, Lactose, Galactose, Mannose oder Xylose. Die N-Alkoxy- oder N-Aryloxy-substituierten Verbindungen können dann durch Umsetzung mit Fettsäuremethylestern in Gegenwart eines Alkoxids als Katalysator in die gewünschten Polyhydroxyfettsäureamide überführt werden.

Der Gehalt an nichtionischen Tensiden beträgt den flüssigen Wasch- und Reinigungsmitteln bevorzugt 5 bis 30 Gew.%, vorzugsweise 7 bis 20 Gew.% und insbesondere 9 bis 15 Gew.%, jeweils bezogen auf das gesamte Mittel.

Als anionische Tenside werden beispielsweise solche vom Typ der Sulfonate und Sulfate eingesetzt. Als Tenside vom Sulfonat-Typ kommen dabei vorzugsweise C9-3-Alkylbenzolsulfonate, Olefinsulfonate, d.h. Gemische aus Alken- und Hydroxyalkansulfonaten sowie Disulfonaten, wie man sie beispielsweise aus C12-8-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C12-8-Alkanen beispielsweise durch Sulfochlorierung oder Sulfoxidation mit anschließender Hydrolyse bzw. Neutralisation gewonnen werden. Ebenso sind auch die Ester von alpha-Sulfofettsäuren (Estersulfonate), zum Beispiel die alpha-sulfonierten Methylester der hydrierten Kokos-, Palmkern- oder Talgfettsäuren geeignet.

Auch geeignet sind Sulfonierungsprodukte von ungesättigten Fettsäuren, beispielsweise Ölsäure, in geringen Mengen, vorzugsweise in Mengen nicht oberhalb etwa 2 bis 3 Gew.-%. Insbesondere sind alpha -Sulfofettsäurealkylester bevorzugt, die eine Alkylkette mit nicht mehr als 4 C-Atomen in der Estergruppe aufweisen, beispielsweise Methylester, Ethylester, Propylester und Butylester. Mit besonderem Vorteil werden die Methylester der alpha - Sulfofettsäuren (MES), aber auch deren verseifte Disalze eingesetzt.

Als weitere anionische Tenside kommen Fettsäure-Derivate von Aminosäuren, beispielsweise von N-Methyltaurin (Tauride) und/oder von N-Methylglycin (Sarkoside) in Betracht. Insbesondere bevorzugt sind dabei die Sarkoside bzw. die Sarkosinate und hier vor allem Sarkosinate von höheren und gegebenenfalls einfach oder mehrfach ungesättigten Fettsäuren wie Oleylsarkosinat.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C12-C18-Fettalkohole, beispielsweise aus Kokosfettalkohol, Talgfettalkohol, Lauryl-, Myristyl-, Cetyl- oder Stearylalkohol oder der C10-C20-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C12-C16-Alkylsulfate und C12-C15-Alkylsulfate sowie C14-C15-Alkylsulfate bevorzugt. Auch 2,3-Alkylsulfate, die beispielsweise als Handelsprodukte der Shell Oil Company unter dem Namen DAN(R) erhalten werden können, sind geeignete Aniontenside.

Auch die Schwefelsäuremonoester der mit 1 bis 6 Mol Ethylenoxid ethoxylierten geradkettigen oder verzweigten C7-21-Alkohole, wie 2-Methyl-verzweigte C9-11-Alkohole mit im Durchschnitt 3,5 Mol Ethylenoxid (EO) oder C12-18-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C8-18-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Insbesondere bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Der Gehalt bevorzugter flüssiger Wasch- und Reinigungsmittel an anionischen Tensiden beträgt 1 bis 30 Gew.%, vorzugsweise 4 bis 25 Gew.% und insbesondere 5 bis 22 Gew.%, jeweils bezogen auf das gesamte Mittel. Es ist besonders bevorzugt, dass die Menge an Fettsäureseife mindestens 2 Gew.%und besonders bevorzugt mindestens 3 Gew.%und insbesondere bevorzugt mindestens 4 Gew.%beträgt.

Als weitere Tenside kommen zur Herstellung der erfindungsgemäßen Wasch- oder Reinigungsmittel sogenannte Gemini-Tenside in Betracht. Hierunter werden im Allgemeinen solche Verbindungen verstanden, die zwei hydrophile Gruppen und zwei hydrophobe Gruppen pro Molekül besitzen. Diese Gruppen sind in der Regel durch einen sogenannten "Spacer" voneinander getrennt. Dieser Spacer ist in der Regel eine Kohlenstoffkette, die lang genug sein sollte, dass die hydrophilen Gruppen einen ausreichenden Abstand haben, damit sie unabhängig voneinander agieren können. Derartige Tenside zeichnen sich im Allgemeinen durch eine ungewöhnlich geringe kritische Micellkonzentration und die Fähigkeit, die Oberflächenspannung des Wassers stark zu reduzieren, aus. In Ausnahmefällen werden jedoch unter dem Ausdruck Gemini-Tenside nicht nur dimere, sondern auch trimere Tenside verstanden.

Gemini-Tenside zur Herstellung von Wasch- oder Reinigungsmitteln sind beispielsweise sulfatierte Hydroxymischether gemäß der deutschen Patentanmeldung DE-A-43 21 022 oder Dimeralkoholbis- und Trimeralkohol-tris-sulfate und -ethersulfate gemäß der deutschen Patentanmeldung DE-A- 195 03 061. Endgruppenverschlossene dimere und trimere Mischether gemäß der deutschen Patentanmeldung DE-A-195 13 391 zeichnen sich insbesondere durch ihre Bi- und Multifunktionalität aus. So besitzen die genannten endgruppenverschlossenen Tenside gute Netzeigenschaften und sind dabei schaumarm, so dass sie sich insbesondere für den Einsatz in maschinellen Wasch- oder Reinigungsverfahren eignen.

Bevorzugt sind aus anwendungstechnischer Sicht Mischungen aus anionischen und nichtionischen Tensiden. Der Gesamt-Tensidgehalt des flüssigen Wasch- und Reinigungsmittel liegt vorzugsweise unterhalb von 40 Gew.- % und besonders bevorzugt unterhalb von 35 Gew.%, bezogen auf das gesamte flüssige Wasch- und Reinigungsmittel.

### A2. Gerüststoffe

Als Gerüststoffe bzw. Builder, die in den flüssigen Wasch- und Reinigungsmitteln enthalten sein können, sind insbesondere Silikate, Aluminiumsilikate (insbesondere Zeolithe), Carbonate, organische Co-builder, Phosphate, Salze organischer Di- und Polycarbonsäuren sowie Mischungen dieser Stoffe zu nennen.

Geeignete kristalline, schichtförmige Natriumsilikate besitzen die allgemeine Formel NaMSiₓO₂ₓ₊₁*H2O, wobei M Natrium oder Wasserstoff bedeutet, x eine Zahl von 1,9 bis 4 und y eine Zahl von 0 bis 20 ist und bevorzugte Werte für x 2, 3 oder 4 sind. Bevorzugte kristalline Schichtsilikate der angegebenen Formel sind solche, in denen M für Natrium steht und x die Werte 2 oder 3 annimmt. Insbesondere sind sowohl beta - als auch delta - Natriumdisilikate Na₂Si₂O₅*yH₂O bevorzugt.

Einsetzbar sind auch amorphe Natriumsilikate mit einem Modul Na₂O: SiO₂ von 1: 2 bis 1: 3,3, vorzugsweise von 1: 2 bis 1: 2,8 und insbesondere von 1: 2 bis 1: 2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, dass die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharte Beugungsmaxima liefern. Dies ist so zu interpretieren, dass die Produkte mikrokristalline Bereiche der Größe 10 bis einige Hundert nm aufweisen, wobei Werte bis maximal 50 nm und insbesondere bis maximal 20 nm bevorzugt sind. Derartige sogenannte röntgenamorphe Silikate, weisen ebenfalls eine Löseverzögerung gegenüber den herkömmlichen Wassergläsern auf. Insbesondere bevorzugt sind verdichtete/kompaktierte amorphe Silikate, compoundierte amorphe Silikate und übertrocknete röntgenamorphe Silikate.

Ein verwendbarer feinkristalliner, synthetischer und gebundenes Wasser enthaltender Zeolith ist vorzugsweise Zeolith A und/oder P. Als Zeolith P wird Zeolith MAP TM (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma SASOL unter dem Markennamen VEGOBOND AX(R) vertrieben wird und durch die Formel

nNa2O*(1-n)K₂O*Al₂O₃.(2-2,5)*SiO₂.(3,5-5,5)*H₂O,

beschrieben werden kann
entspricht. Der Zeolith kann als sprühgetrocknetes Pulver oder auch als ungetrocknete, von ihrer Herstellung noch feuchte, stabilisierte Suspension zum Einsatz kommen. Für den Fall, dass der Zeolith als Suspension eingesetzt wird, kann diese geringe Zusätze an nichtionischen Tensiden als Stabilisatoren enthalten, beispielsweise 1 bis 3 Gew.%, bezogen auf Zeolith, an ethoxylierten C₁₂-C₁₈-Fettalkoholen mit 2 bis 5 Ethylenoxidgruppen, C₁₂-C₁₄-Fettalkoholen mit 4 bis 5 Ethylenoxidgruppen oder ethoxylierten Isotridecanolen. Geeignete Zeolithe weisen eine mittlere Teilchengröße von weniger als 10 µm (Volumenverteilung; Messmethode: Coulter Counter) auf und enthalten vorzugsweise 18 bis 22 Gew.%, insbesondere 20 bis 22 Gew.% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate.

Als Builder sind organische Co-builder geeignet, insbesondere Polycarboxylate/Polycarbonsäuren, polymere Polycarboxylate, Asparaginsäure, Polyacetale, Dextrine, sowie Phosphonate.

Polymere Polycarboxylate sind beispielsweise die Alkalimetallsalze der Polyacrylsäure oder der Polymethacrylsäure, beispielsweise solche mit einer relativen Molekülmasse von 500 bis 70000 g/mol. Bei den für polymere Polycarboxylate angegebenen Molmassen handelt es sich im Sinne dieser Schrift um gewichtsmittlere Molmassen Mw der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsaeure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2.000 bis 70.000 g/mol, vorzugsweise 20.000 bis 50.000 g/mol und insbesondere 30.000 bis 40.000 g/mol.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder die als Monomere Salze der Acrylsäure und der 2-Alkylallylsulfonsaeure sowie Zucker-Derivate enthalten.

Weitere bevorzugte Copolymere sind solche, die als Monomere vorzugsweise Acrolein und Acrylsäure/Acrylsäuresalze bzw. Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, die neben Co-builder-Eigenschaften auch eine bleichstabilisierende Wirkung aufweisen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500.000 g/mol. Dabei ist ein Polysaccharid mit einem Dextrose- äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weissdextrine mit höheren Molmassen im Bereich von 2000 bis 30000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Ein an C6 des Saccharidrings oxidiertes Produkt kann besonders vorteilhaft sein.

Ein bevorzugtes Dextrin ist in der britischen Patentanmeldung GB 9,419,091 B1 beschrieben. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Derartige oxidierte Dextrine und Verfahren ihrer Herstellung sind beispielsweise aus den europäischen Patentanmeldungen EP 032202 A**,** EP 0427349 A**,** EP 0472042 A und EP 0542496 A sowie den internationalen Patentanmeldungen WO 1992/018542 A**,** WO 1993/008251 A**,** WO 1994/ 028030 A**,** WO 1995/007303 A**,** WO 1995/012619 A und WO 1995/020608 A bekannt. Ein an C₆ des Saccharidrings oxidiertes Produkt kann besonders vorteilhaft sein.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate, wie sie beispielsweise in den US-amerikanischen Patentschriften US 4,524,009**,** US 4,639 325**,** in der europäischen Patentanmeldung EP 0150930 A und der japanischen Patentanmeldung JP 1993/339896 A beschrieben werden.

Weitere brauchbare organische Co-builder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten. Derartige Co-builder werden beispielsweise in der internationalen Patentanmeldung WO 1995/020029 A beschrieben.

Eine weitere Substanzklasse mit Co-buildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Co-builder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Wasch- und Reinigungsmittel auch Bleiche enthalten, bevorzugt sein Aminoalkan-phosphonate, insbesondere DTPMP, einzusetzen, oder Mischungen aus den genannten Phosphonaten zur Herstellung der Mittel zu verwenden.

Darüber hinaus können alle Verbindungen, die in der Lage sind, Komplexe mit Erdalkaliionen auszubilden, als Co-builder eingesetzt werden.

Weitere brauchbare organische Gerüstsubstanzen sind auch die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes von Wasch- und/oder Reinigungsmitteln. Insbesondere sind hierbei Citronensäure, Bernsteinsäure, Glutarsäure, Adipinsäure, Gluconsäure und beliebige Mischungen aus diesen zu nennen.

### A3. Bleichmittel und Bleichkatalysatoren

Unter den als Bleichmitteln dienenden, in Wasser H2O2 liefernden Verbindungen haben das Natriumperborattetrahydrat und das Natriumperboratmonohydrat besondere Bedeutung. Weitere brauchbare Bleichmittel sind beispielsweise Natriumpercarbonat, Peroxypyrophosphate, Citratperhydrate sowie H2O2 liefernde persaure Salze oder Persäuren, wie Perbenzoate, Peroxophthalate, Diperazelainsäure, Phthaloiminopersäure oder Diperdodecandisäure. Um beim Waschen bei Temperaturen von 60 °C und darunter eine verbesserte Bleichwirkung zu erreichen, können Bleichaktivatoren in die Wasch- und Reinigungsmittel eingearbeitet werden. Als Bleichaktivatoren können Verbindungen, die unter Perhydrolysebedingungen aliphatische Peroxocarbonsäuren mit vorzugsweise 1bis 10 C-Atomen, insbesondere 2 bis 4 C-Atomen, und/oder gegebenenfalls substituierte Perbenzoesäure ergeben, eingesetzt werden. Geeignet sind Substanzen, die O-und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin(DADHT), acylierte Glykolurile, insbesondere Tetraacetylglykoluril (TAGU), N-Acylimide,insbesondere N-Nonanoylsuccinimid (NOSI), acylierte Phenolsulfonate, insbesonderen-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran. Zusätzlich zu den konventionellen Bleichaktivatoren oder an deren Stelle können auch sogenannte Bleichkatalysatoren in die Textilbehandlungsmitteleingearbeitet werden. Bei diesen Stoffen handelt es sich um bleichverstärkende Übergangsmetallsalze bzw. Übergangsmetallkomplexe wie beispielsweise Mn-,Fe-, Co-, Ru- oder Mo-Salenkomplexe oder -carbonylkomplexe. Auch Mn-, Fe-, Co-,Ru-, Mo-, Ti-, V- und Cu-Komplexe mit stickstoffhaltigen Tripod-Liganden sowie Co-, Fe-, Cu- und Ru-Amminkomplexe sind als Bleichkatalysatoren verwendbar.

### A4. Verdickungsmittel

Ein flüssiges Wasch- und Reinigungsmittel kann ein Verdickungsmittelenthalten. Das Verdickungsmittel kann beispielsweise einen Polyacrylat-Verdicker, Xanthan Gum, Gellan Gum, Guarkernmehl, Alginat, Carrageenan, Carboxymethylcellulose, Bentonite, Wellan Gum, Johannisbrotkernmehl, Agar-Agar, Tragant, Gummi arabicum, Pektine, Polyosen, Stärke, Dextrine, Gelatine und Casein umfassen. Aber auch abgewandelte Naturstoffe wie modifizierten Stärken und Cellulosen, beispielhaftseien hier Carboxymethylcellulose und andere Celluloseether, Hydroxyethyl- und -propylcellulose sowie Kernmehlether genannt, können als Verdickungsmittel eingesetzt werden.

Zu den Polyacryl- und Polymethacryl-Verdickern zählen beispielsweise die hochmolekularen mit einem Polyalkenylpolyether, insbesondere einem Allylether von Saccharose, Pentaerythrit oder Propylen, vernetzten Homopolymere der Acrylsäure (INCI-Bezeichnung gemäß "International Dictionary of Cosmetic Ingredients "der "The Cosmetic, Toiletry and Fragrance Association (CTFA)": Carbomer),die auch als Carboxyvinylpolymere bezeichnet werden. Solche Polyacrylsäuren sind u.a. von der Fa. 3V Sigma unter dem Handelsnamen Polygel®,z.B. Polygel DA, und von der Fa. B.F. Goodrich unter dem Handelsnamen Carbopol® erhältlich, z.B. Carbopol 940 (Molekulargewicht ca. 4.000.000), Carbopol 941 (Molekulargewicht ca. 1.250.000) oder Carbopol 934 (Molekulargewicht ca. 3.000.000).Weiterhin fallen darunter folgende Acrylsäure-Copolymere: (i) Copolymere von zwei oder mehr Monomeren aus der Gruppe der Acrylsäure, Methacrylsäure und ihrer einfachen, vorzugsweise mit C1-4-Alkanolen gebildeten, Ester (INCI AcrylatesCopolymer), zu denen etwa die Copolymere von Methacrylsäure, Butylacrylat und Methylmethacrylat (CAS-Bezeichnung gemäß Chemical Abstracts Service: 25035-69-2)oder von Butylacrylat und Methylmethacrylat (CAS 25852-37-3) gehören und die beispielsweise von der Fa. Rohm and Haas unter den Handelsnamen Aculyn® und Acusol® sowie von der Firma Degussa (Goldschmidt) unter dem Handelsnamen Tego® Polymer erhältlich sind, z.B. die anionischen nicht assoziativen Polymere Aculyn 22, Aculyn 28, Aculyn 33 (vernetzt), Acusol 810,Acusol 820, Acusol 823 und Acusol 830 (CAS 25852-37-3); (ii) vernetzte hochmolekulare Acrylsäurecopolymere, zu denen etwa die mit einem Allylether der Saccharose oder des Pentaerythrits vernetzten Copolymere von C10-30-Alkylacrylaten mit einem oder mehreren Monomeren aus der Gruppe der Acrylsäure, Methacrylsäure und ihrer einfachen, vorzugsweise mit C1-4-Alkanolen gebildeten, Ester (INCI Acrylates/C10-30Alkyl Acrylate Crosspolymer) gehören und die beispielsweise von der Fa. B.F. Goodrich unter dem Handelsnamen Carbopol® erhältlich sind, z.B. das hydrophobierte Carbopol ETD 2623 und Carbopol 1382 (INCI Acrylates/C10-30Alkyl Acrylate Crosspolymer) sowie Carbopol Aqua 30 (früher Carbopol EX 473).

Ein weiteres bevorzugt einzusetzendes polymeres Verdickungsmittel ist Xanthan Gum, ein mikrobielles anionisches Heteropolysaccharid, das von Xanthomonascampestris und einigen anderen Species unter aeroben Bedingungen produziert wird und eine Molmasse von 2 bis 15 Millionen Dalton aufweist. Xanthan wird aus einer Kette mit beta-1,4-gebundener Glucose (Cellulose) mit Seitenketten gebildet. Die Struktur der Untergruppen besteht aus Glucose, Mannose, Glucuronsaeure,Acetat und Pyruvat, wobei die Anzahl der Pyruvat-Einheiten die Viskosität des Xanthan Gums bestimmt. Als Verdickungsmittel kommt insbesondere auch ein Fettalkohol in Frage. Fettalkohole können verzweigt oder nichtverzweigt sowie nativen Ursprungs oderpetrochemischen Ursprungs sein. Bevorzugte Fettalkohole haben eine C-Kettenlänge von 10 bis 20 C-Atomen, bevorzugt 12 bis 18. Bevorzugt werden Mischungen unterschiedlicher C-Kettenlängen, wie Talgfettalkohol oder Kokosfettalkohol, eingesetzt. Beispiele sind Lorol® Spezial (C12-14-ROH) oder Lorol® Technisch(C12-18-ROH) (beide ex Cognis). Bevorzugte flüssige Wasch- und Reinigungsmittel enthalten bezogen auf das gesamte Mittel 0.01 bis 3 Gew.% und vorzugsweise 0.1 bis1 Gew.% Verdickungsmittel. Die Menge an eingesetztem Verdickungsmittel ist dabei abhängig von der Art des Verdickungsmittels und dem gewünschten Grad der Verdickung.

### A5. Enzyme

Die Wasch- und Reinigungsmitteln können Enzyme in verkapselter Form und/oder direkt in den Wasch- und Reinigungsmittel enthalten. Als Enzyme kommen insbesondere solche aus der Klassen der Hydrolasen wie der Proteasen, Esterasen, Lipasen bzw. lipolytisch wirkende Enzyme, Amylasen, Cellulasen bzw. andere Glykosylhydrolasen, Hemicellulase, Cutinasen, beta-Glucanasen, Oxidasen, Peroxidasen, Perhydrolasen und/oder Laccasen und Gemische der genannten Enzyme in Frage. Alle diese Hydrolasen tragen in der Wäsche zur Entfernung von Verfleckungen wie Protein-, fett- oder stärkehaltigen Verfleckungen und Vergrauungen bei. Cellulasen und andere Glykosylhydrolasenkoennen darüber hinaus durch das Entfernen von Pilling und Mikrofibrillen zur Farberhaltung und zur Erhöhung der Weichheit des Textils beitragen. Zur Bleiche bzw. zur Hemmung der Farbübertragung können auch Oxireduktasen eingesetzt werden. Besonders gut geeignet sind aus Bakterienstämmen oder Pilzen wie Bacillus subtilis, Bacillus licheniformis, Streptomyceus griseus und Humicolainsolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere alpha-Amylasen, Iso-Amylasen, Pullulanasen und Pektinasen. Als Cellulasen werden vorzugsweise Cellobiohydrolasen, Endoglucanasen und p-Glucosidasen, die auch Cellobiasen genannt werden, bzw. Mischungen aus diesen eingesetzt. Da sich verschiedene Cellulase-Typen durch ihre CMCase- und Avicelase-Aktivitäten unterscheiden, können durch gezielte Mischungen der Cellulasen die gewünschten Aktivitäten eingestellt werden.

Die Enzyme können an Trägerstoffe adsorbiert sein, um sie gegen vorzeitige Zersetzung zu schützen. Der Anteil der Enzyme, der Enzymflüssigformulierung(en) oder der Enzymgranulate direkt in Wasch- und Reinigungsmittel kann beispielsweise etwa 0,01 bis 5 Gew.%, vorzugsweise 0.12 bis etwa 2.5 Gew.% betragen.

Es kann, beispielsweise bei speziellen Wasch- und Reinigungsmitteln für Konsumenten mit Allergien, aber auch bevorzugt sein, dass das Wasch- und Reinigungsmittel keine Enzyme enthält.

### A6. Elektrolyte

Als Elektrolyte aus der Gruppe der anorganischen Salze kann eine breite Anzahl der verschiedensten Salze eingesetzt werden. Bevorzugte Kationen sind die Alkali- und Erdalkalimetalle, bevorzugte Anionen sind die Halogenide und Sulfate. Aus herstellungstechnischer Sicht ist der Einsatz von NaCl oder MgCl₂ in den Wasch- und Reinigungsmitteln bevorzugt. Der Anteil an Elektrolyten in den Wasch- und Reinigungsmittel beträgt üblicherweise 0,1 bis 5 Gew.-%.

### A7. Lösungsmittel

Nichtwässrige Lösungsmittel, die in den flüssigen Wasch- und Reinigungsmittel eingesetzt werden können, stammen beispielsweise aus der Gruppe der ein- oder mehrwertigen Alkohole, Alkanolamine oder Glykolether, sofern sie im angegebenen Konzentrationsbereich mit Wasser mischbar sind. Vorzugsweise werden die Lösungsmittel ausgewählt aus Ethanol, n- oder i-Propanol, Butanolen, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethyl-, -ethyl-oder -propylether, Dipropylenglykolmonomethyl- oder -ethylether, Di-isopropylenglykolmonomethyl-oder -ethylether, Methoxy-, Ethoxy- oder Butoxytriglykol, 1-Butoxyethoxy-2-propanol,3-Methyl-3-methoxybutanol, Propylen-glykol-t-butylether sowie Mischungen dieser Lösungsmittel. Nichtwässrige Lösungsmittel können in den flüssigen Wasch- und Reinigungsmitteln in Mengen zwischen 0,5 und 15 Gew.%, bevorzugt aber unter 12 Gew.% und insbesondere unterhalb von 9 Gew.% eingesetzt werden.

### A8. Viskosität

Die Viskosität der Wasch- und Reinigungsmittel in flüssiger Form kann mit üblichen Standardmethoden (beispielsweise Brookfield-Viskosimeter LVT-II bei 20 U/min und 20°C, Spindel 3) gemessen werden und liegt für flüssige Waschmittel vorzugsweise im Bereich von 500 bis 5000 mPas. Bevorzugte weisen flüssige Wasch- und Reinigungsmitteln Viskositäten von 700 bis 4000 mPas auf, wobei Werte zwischen 1000 und 3000 mPas besonders bevorzugt sind. Die Viskosität von Weichspülern beträgt vorzugsweise 20 bis 4000 mPas, wobei Werte zwischen 40 und 2000 mPas besonders bevorzugt sind. Insbesondere bevorzugt liegt die Viskosität von Weichspülern von 40 bis 1000 mPas.

### A9. pH-Stellmittel

Um den pH-Wert der flüssigen Wasch- und Reinigungsmitteln in den gewünschten Bereich zu bringen, kann der Einsatz von pH-Stellmitteln angezeigt sein. Einsetzbar sind hier sämtliche bekannten Säuren bzw. Laugen, sofern sich ihr Einsatz nicht aus anwendungstechnischen oder ökologischen Gründen bzw. aus Gründen des Verbraucherschutzes verbietet. Üblicherweise überschreitet die Menge dieser Stellmittel 7 Gew.% der Gesamtformulierung nicht. Der pH-Wert von flüssigen Wasch- und Reinigungsmitteln liegt bevorzugt zwischen 4 und 10 und bevorzugt zwischen 5.5 und 8.5. Der pH-Wert von flüssigen Weichspülern liegt bevorzugt zwischen 1 und 6 und bevorzugt zwischen 1.5 und 3.5.

### A10. Farbstoffe

Um den ästhetischen Eindruck der Textilbehandlungsmittel zu verbessern, können sie mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Wasch- und Reinigungsmittel und gegen Licht sowie keine ausgeprägte Substantivität gegenüber Textilfasern, um diese nicht anzufärben.

### A11. Antiredepositionsmittel

Geeignete Soil-Release-Polymere, die auch als "Antiredepositionsmittel" bezeichnet werden, sind beispielsweise nichtionische Celluloseether wie Methylcellulose und Methylhydroxypropylcellulose mit einem Anteil an Methoxygruppen von 15 bis 30Gew.% und an Hydroxypropylgruppen von 1 bis 15 Gew.%, jeweils bezogen auf den nichtionischen Celluloseether sowie die aus dem Stand der Technik bekannten Polymere der Phthalsäure und/oder Terephthalsäure bzw. von deren Derivaten, insbesondere Polymere aus Ethylenterephthalaten und/oder Polyethylen- und/oder Polypropylenglykolterephthalaten oder anionisch und/oder nichtionisch modifizierten Derivaten von diesen. Geeignete Derivate umfassen die sulfonierten Derivate der Phthalsäure- und Terephthalsäure-Polymere.

### A12. Optische Aufheller

Optische Aufheller (sog. "Weisstöner") können den Wasch- und Reinigungsmitteln zugesetzt werden, um Vergrauungen und Vergilbungen der behandelten Textilen Flächengebilden zu beseitigen. Diese Stoffe ziehen auf die Faser auf und bewirken eine Aufhellung und vorgetäuschte Bleichwirkung, indem sie unsichtbare Ultraviolettstrahlung in sichtbares längenwelliges Lichtumwandeln, wobei das aus dem Sonnenlicht absorbierte ultraviolette Licht als schwach bläuliche Fluoreszenz abgestrahlt wird und mit dem Gelbton der vergrauten bzw. vergilbten Wäsche reines Weiss ergibt. Geeignete Verbindungen stammen beispielsweiseaus den Substanzklassen der 4,4'-Diamino-2,2'-stilbendisulfonsaeuren (Flavonsäuren), 4,4'-Distyryl-biphenylen, Methylumbelliferone, Cumarine, Dihydrochinolinone, 1,3-Diarylpyrazoline,Naphthalsaeureimide, Benzoxazol-, Benzisoxazol- und Benzimidazol-Systeme sowie der durch Heterocyclen substituierten Pyrenderivate. Die optischen Aufheller werden üblicherweise in Mengen zwischen 0 % und 0.3 Gew.%, bezogen auf das fertige Wasch- und Reinigungsmittel, eingesetzt.

### A13. Vergrauungsinhibitoren

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Naturgeeignet, beispielsweise Leim, Gelatine, Salze von Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, zum Beispiel abgebaute Stärke, Aldehydstärken usw. Auch Polyvinylpyrrolidon ist brauchbar. Bevorzugt werden jedoch Celluloseether wie Carboxymethylcellulose (Na-Salz), Methylcellulose, Hydroxyalkylcellulose und Mischether wie Methylhydroxyethylcellulose, Methylhydroxypropy-Icellulose, Methylcarboxymethyl-cellulose und deren Gemische in Mengen von 0.1 bis 5 Gew.%, bezogen auf die Wasch- und Reinigungsmitteln, eingesetzt.

### A14. Knitterschutzmittel

Da textile Flächengebilde, insbesondere aus Reyon, Zellwolle, Baumwolle und deren Mischungen, zum Knittern neigen können, weil die Einzelfasern gegen Durchbiegen, Knicken, Pressen und Quetschen quer zur Faserrichtung empfindlich sind, können die Wasch- und Reinigungsmittel synthetische Knitterschutzmittel enthalten. Hierzu zählen beispielsweise synthetische Produkte auf der Basis von Fettsäuren, Fettsäureestern, Fettsäureamiden, - alkylolestern,-alkylolamiden oder Fettalkoholen, die meist mit Ethylenoxid umgesetzt sind, oder Produkte auf der Basis von Lecithin oder modifizierter Phosphorsäureester.

### A15. Antimikrobielle Wirkstoffe

Zur Bekämpfung von Mikroorganismen können die Wasch- und Reinigungsmitteln antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppensind beispielsweise Benzalkoniumchloride, Alkylarylsulfonate, Halogenphenole und Phenolmercuriacetat, wobei bei den erfindungemäßen Wasch- und Reinigungsmitteln auch gänzlich auf diese Verbindungen verzichtet werden kann.

### A16. Konservierungsmittel

Die erfindungsgemäßen Wasch- und Reinigungsmittel können Konservierungsmittel enthalten, wobei vorzugsweise nur solche eingesetzt werden, die kein oder nur ein geringes hautsensibilisierendes Potential besitzen. Beispiele sind Sorbinsäure und seine Salze, Benzoesäure und seine Salze, Salicylsäure und seine Salze, Phenoxyethanol, 3-lodo-2-propynylbutylcarbamat, Natrium N-(hydroxymethyl)glycinat, Biphenyl-2-ol sowie Mischungen davon. Ein geeignetes Konservierungsmittel stellt die lösungsmittelfreie, wässrige Kombination von Diazolidinylharnstoff, Natriumbenzoat und Kaliumsorbat (erhältlich als Euxyl® K 500ex Schülke and Mayr) dar, welches in einem pH-Bereich bis 7 eingesetzt werden kann. Insbesondere eignen sich Konservierungsmittel auf Basis von organischen Säuren und/oder deren Salzen zur Konservierung der erfindungsgemäßen, hautfreundlichen Wasch- und Reinigungsmittel.

### A17. Antioxidantien

Um unerwünschte, durch Sauerstoffeinwirkung und andere oxidative Prozesse verursachte Veränderungen an den Wasch- und Reinigungsmitteln und/oder den behandelten textilen Flächengebilden zu verhindern, können die Wasch- und Reinigungsmittel Antioxidantien enthalten. Zu dieser Verbindungsklasse gehören beispielsweise substituierte Phenole, Hydrochinone, Brenzcatechine und aromatische Amine sowie organische Sulfide, Polysulfide, Dithiocarbamate, Phosphite, Phosphonate und Vitamin E.

### A18. Antistatika

Ein erhöhter Tragekomfort kann aus der zusätzlichen Verwendung von Antistatika resultieren, die den Wasch- und Reinigungsmitteln zusätzlich beigefügt werden. Antistatika vergrößern die Oberflächenleitfähigkeit und ermöglichen damit ein verbessertes Abfließen gebildeter Ladungen. Antistatika sind in der Regel Substanzen mit wenigstens einem hydrophilen Molekuelliganden und geben auf den Oberflächen einen mehr oder minder hygroskopischen Film. Diese zumeist grenzflächenaktiven Antistatika lassen sich in stickstoffhaltige (Amine, Amide, quartäre Ammoniumverbindungen), phosphorhaltige (Phosphorsaeureester) und schwefelhaltige (Alkylsulfonate, Alkylsulfate) Antistatika unterteilen. Lauryl-(bzw. Stearyl-)dimethylbenzylammoniumchloride eignen sich als Antistatika für textile Flächengebilde bzw. als Zusatz zu Wasch- und Reinigungsmitteln, wobei zusätzlich ein Avivageeffekt erzielt wird.

### A19. Schauminhibitoren

Zur Verbesserung der Wiederbenetzbarkeit der behandelten textilen Flächengebilde und zur Erleichterung des Bügelns der behandelten textilen Flächengebilde können in den Textilbehandlungsmitteln beispielsweise Silikonderivate eingesetzt werden. Diese verbessern zusätzlich das Ausspülverhalten der Wasch- und Reinigungsmittel durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind bzw. Si-OH-, Si-H- und/oder Si-Cl-Bindungen aufweisen. Die Viskositäten der bevorzugten Silikone liegen bei 25 °C im Bereich zwischen 100 und 100.000 mPas, wobei die Silikone in Mengen zwischen 0.2 und 5 Gew.%, bezogen auf das gesamte Wasch- und Reinigungsmittel eingesetzt werden können.

### A20. UV-Absorber

Schließlich können die Wasch- und Reinigungsmittel auch UV-Absorber enthalten, die auf die behandelten textilen Flächengebilde aufziehen und die Lichtbeständigkeit der Fasern verbessern. Verbindungen, die diese gewünschten Eigenschaften aufweisen, sind beispielsweise die durch strahlungslose Deaktivierung wirksamen Verbindungen und Derivate des Benzophenons mit Substituenten in 2- und/oder4-Stellung. Weiterhin sind auch substituierte Benzotriazole, in 3-Stellung Phenyl-substituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet.

### A21. Schwermetallkomplexbildner

Um die durch Schwermetalle katalysierte Zersetzung bestimmter Waschmittel-Inhaltsstoffe zu vermeiden, können Stoffe eingesetzt werden, die Schwermetalle komplexieren. Geeignete Schwermetallkomplexbildner sind beispielsweise die Alkalisalze der Ethylendiamintetraessigsäure(EDTA) oder der Nitrilotriessigsäure (NTA) sowie Alkalimetallsalze von anionischen Polyelektrolyten wie Polymaleaten und Polysulfonaten. Eine bevorzugte Klasse von Komplexbildnern sind die Phosphonate, die in bevorzugten Textilbehandlungsmitteln in Mengen von 0.01 bis 2.5 Gew.%, vorzugsweise 0.02 bis 2 Gew.% und insbesondere von 0.03 bis 1.5 Gew.% enthalten sind. Zu diesen bevorzugten Verbindungen zählen insbesondere Organophosphonate wie beispielsweisel-Hydroxyethan-1,1-diphosphonsaeure (HEDP), Aminotri(methylenphosphonsäure) (ATMP), Diethylentriamin-penta (methylenphosphonsäure) (DTPMP bzw. DETPMP) sowie 2-Phosphonobutan-1,2,4-tricarbonsäure (PBS-AM), die zumeist in Form ihrer Ammonium- oder Alkalimetallsalze eingesetzt werden.

### A23. Herstellung der Zubereitungen

Die Herstellung der flüssigen Wasch-, Spül- und Reinigungsmittel erfolgt mittels üblicher und bekannter Methoden und Verfahren in dem beispielsweise die Bestandteile einfach in Rührkesseln vermischt werden, wobei Wasser, nicht-wässrige Lösungsmittel und Tenside, zweckmäßigerweise vorgelegt werden und die weiteren Bestandteile portionsweise hinzugefügt werden. So können flüssige Wasch- und Reinigungsmittel hergestellt werden, indem die sauren Komponenten wie beispielsweise die linearen Alkylsulfonate, Zitronensäure, Borsäure, Phosphonsäure, die Fettalkoholethersulfate, usw. und die nichtionischen Tenside vorgelegt werden. Die Lösungsmittelkomponente wird vorzugsweise auch zu diesem Zeitpunkt hinzugegeben, die Zugabe kann aber auch zu einem späteren Zeitpunkt erfolgen. Zu diesen Komponenten wird das Polyacrylat gegeben. Anschließend wird eine Base wie beispielsweise NaOH, KOH, Triethanolamin oder Monoethanolamin gefolgt von der Fettsäure, falls vorhanden, zugegeben. Darauf folgend werden die restlichen Inhaltsstoffe und die restlichen Lösungsmittel des wässrigen flüssigen Wasch- und Reinigungsmittel zu der Mischung gegeben und der pH-Wert auf ungefähr 8,5 eingestellt. Abschließend können die zu dispergierenden Partikel zugegeben und durch Mischen homogen in dem wässrigen flüssigen Wasch- und Reinigungsmittel verteilt werden.

Bei den erfindungemäßen Mitteln handelt es sich vorzugsweise um Waschmittel, die sowohl für manuelle oder maschinelle Wäsche, insbesondere von Textilien geeignet sind. Es kann sich auch um Wasch- oder Reinigungsmittel für den industriellen Bereich oder für den Haushaltsbereich handeln. Reinigungsmittel können beispielsweise auch zur Reinigung harter Oberflächen eingesetzt werden. Es kann sich dabei beispielsweise um Geschirrreinigungsmittel handeln, die zur manuellen oder maschinellen Reinigung von Geschirr eingesetzt werden. Es kann sich auch um übliche Industrie- oder Haushaltsreiniger handeln, mit denen harte Oberflächen wie Möbeloberflächen, Fliesen, Kacheln, Wand- und Bodenbeläge gereinigt werden. Als harte Oberflächen kommen neben Geschirr auch alle übrigen harten Oberflächen, insbesondere aus Glas, Keramik, Kunststoff oder Metall, in Haushalt und Gewerbe in Frage.

Dabei handelt es sich bei den Wasch- und Reinigungsmitteln bevorzugt um flüssige Formulierungen, wobei es sich um Lösungen, Emulsionen, Dispersionen Suspensionen, Mikroemulsionen, Gels oder Pasten handeln kann.

Als Oberflächenbehandlungsmittel kann das Mittel dementsprechend übliche Inhaltsstoffe von Reinigungsmitteln in üblichen Mengen enthalten. Beispielsweise können Oberflächenbehandlungsmittel als Reinigungsmittel, Alkylethersulfate, Alkyl- und/oder Arylsulfonate, Alkylsulfate, Amphotenside, Anionentenside, nichtionische Tenside, kationische Tenside, Lösungsmittel, Verdickungsmittel, Dicarbonsäure (salze) und weitere Hilfs- und Zusatzstoffe enthalten.

Diese zusätzlichen Inhaltstoffe sind teilweise bereits vorne näher erläutert und gelten hier ebenfalls für den Einsatz in Reinigungsmittel(siehe z.B. nichtionische Tenside). Als Hilfs- und Zusatzstoffe können - insbesondere in Handgeschirrspülmitteln und Reinigungsmitteln für harte Oberflächen, insbesondere UV-Stabilisatoren, Parfüm, Perlglanzmittel (INCI Opacifying Agents; beispielsweise Glykoldistearat, z.B. Cutina® AGS der Fa. Henkel KGaA, bzw. dieses enthaltende Mischungen, z.B. die Euperlane® der Fa. Henkel KGaA), SRP (soil repellent polymers), PEG-Terephthalate, Farbstoffe, Bleichmittel (z. B. Wasserstoffperoxid), Korrosionsinhibitoren, Konservierungsmittel (z.B. das technische auch als Bronopol bezeichnete 2-Brom-2-nitropropan-1,3-diol(CAS 52-51-7), das beispielsweise als Myacide® BT oder als BootsBronopol BT von der Firma Boots gewerblich erhältlich ist) sowie Hautgefühlverbessernde oder pflegende Additive (z.B. dermatologisch wirksame Substanzen wie Vitamin A, Vitamin B2, Vitamin B12, Vitamin C, Vitamin E, D-Panthenol, Sericerin, Collagen-Partial-Hydrolysat,verschiedene pflanzliche Protein-Partial-Hydrolysate, Proteinhydrolysat-Fettsäure-Kondensate, Liposome, Polypropylenglykol, Nutrilan™ Chitosan™, Cholesterin, pflanzliche und tierische Öle wie z.B. Lecithin, Sojaöl, usw., Pflanzenextrakte wie z.B. Aloe Vera, Azulen, Hamamelisextrakte, Algenextrakte, usw., Allantoin, A.H.A.-Komplexe, in Mengen von üblicherweise nicht mehr als 5 Gew.% enthalten sein. Zur Leistungssteigerung können geringe Mengen Enzyme eingesetzt werden. Bevorzugt sind Proteasen (z.B. BLAP (Henkel), Savinase (NOVO), Durazym (NOVO), Maxapemm, etc.), Amylasen (z.B. Fermamyl (NOVO), etc.), Lipasen (z.B. Lipolase (NOVO), etc.), Peroxidasen, Gluconasen, Cellulasen, Mannasen, usw., in Mengen von vorzugsweise 0.001 bis 1.5 Gew.% und besonders bevorzugt weniger als 0.5 Gew.%.

### Kosmetische Zubereitungen

Ein weiterer Gegenstand der Erfindung betrifft kosmetische Zubereitungen, enthaltend mindestens ein Lacton der Formel (I), wobei die Einsatzkonzentration des Lactons oder der Lactone üblicherweise im Bereich von 0,01 bis 1 Gew.-%, vorzugsweise 0,1 bis 0,5 Gew.-% - bezogen auf die Zubereitungen - beträgt.

Die erfindungsgemäßen kosmetischen Mittel können weitere typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### B1. Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionisehe Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### B2. Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### B3. Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia® SP von Cognis;
- Polyalkylenglycole sowie
- Glycerincarbonat.

Im Folgenden werden besonders geeignete Emulgatoren näher erläutert:
(i) **Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.
(ii) **Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.
(iii) **Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.
(iv) **Sorbitanester.** Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.
(v) **Polyglycerinester.** Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.
(vi) **Anionische Emulgatoren.** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.
(vii) **Amphotere und kationische Emulgatoren.** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### B4. Fette und Wachse

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### B5. Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### B6. Kühlstoffe

Kühlstoffe sind Verbindungen, die auf der Haut ein Gefühlt der Kälte erzeugen. In der Regel handelt es sich dabei um Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.
¹ FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:

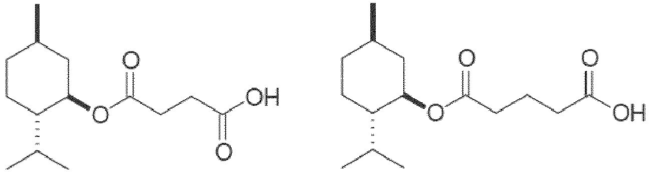

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat® MGC), Menthol Propylenglycol Carbonate (FEMA GRAS 3784 = Frescolat® MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat® ML) und insbesondere das Menthone Glyceryl Acetal (FEMA GRAS 3807) bzw. Menthone Glyceryl Ketal (FEMA GRAS 3808), das unter der Bezeichnung Frescolat® MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat® MGA, Frescolat® ML, Frecolat® MGC und Frescolat® MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

### B7. Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und - diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### B8. Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

### B9. Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### B10. Silikonverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### B11. UV-Lichtschutzfaktoren

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-iso-propylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan® AP)
- Sulfonsäurederivate von Benzophenonen, vorzugsweise 2-Hydroxy-4-methoxybenzo-phenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 2-(4-Diethylamino-2-hydroxybenzoyl)-benzoic acid hexylester (Uvinul® A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000, Eusolex® T, Eusolex® T-ECO, Eusolex® T-S, Eusolex® T-Aqua, Eusolex® T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE® oder Z-COTE HP1® verwendet.

### B12. Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### B13. Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis (µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. VitaminE-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### B14. Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.
(i) **Keimhemmende Mittel.** Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.
(ii) **Enzyminhibitoren.** Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.
(iii) **Geruchsabsorber.** Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.
(iv) **Antitranspirantien.** Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
   - adstringierende Wirkstoffe,
   - Ölkomponenten,
   - nichtionische Emulgatoren,
   - Coemulgatoren,
   - Konsistenzgeber,
   - Hilfsstoffe wie z. B. Verdicker oder Komplexierungsmittel und/oder
   - nichtwässrige Lösungsmittel wie z. B. Ethanol, Propylenglykol und/oder Glycerin. Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichloro-hydrat, Aluminium-Zirkoniumtetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
   - entzündungshemmende, hautschützende oder wohlriechende ätherische Öle,
   - synthetische hautschützende Wirkstoffe und/oder
   - öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### B15. Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### B16. Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### B17. Quellmittel

Als Quellmittel für wässrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden.

### B18. Insektenrepellentien

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### B19. Inhaltsstoffe für Mund- und Zahnpflegemittel

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxidtrihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

### B20. Hydrotrope

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### B21. Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### B22. Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### B23. Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.1.42051), Indigotin (C.1.73015), Chlorophyllin (C.1.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Mischungen

Ein weiterer Gegenstand der Erfindung betrifft die Verwendung von Mischungen, enthaltend weitere Lactone ausgewählt aus der Gruppe, die gebildet wird von γ-Hexalacton, γ-Heptalacton, γ-Octalacton, γ-Nonalacton, γ-Dodecalacton, δ-Hexalacton, δ-Heptalacton, δ-Octalacton, δ-Nonalacton, δ-Decalacton, δ-Undecalacton, δ-Dodecalacton sowie deren Gemischen.

Es hat sich nämlich erwiesen, dass sich die besten geruchlichen Verbesserungen erzielen lassen, wenn man insbesondere drei oder vier Lactone gemeinsam einsetzt, wobei das Gewichtsverhältnis in etwa ausgeglichen sein sollte.

Die Mischungen können den Endprodukten in den bereits genannten Mengen, als etwa 0,01 bis 1 Gew.-%, insbesondere 0,02 bis 0,5 Gew.-% und besonders bevorzugt 0,05 bis 0,1 Gew.-% - bezogen auf die Zubereitungen - zugegeben werden.

Ein weiterer Gegenstand der Erfindung ist auf Mischungen gerichtet, die
(a) γ-Decalacton,
(b) γ-Undecalacton und
(c) Aldehyde der Formel (II)

   R²CHO (II)
enthalten, wobei R² für einen linearen oder verzweigten, gesättigten oder ungesättigten Alkyl- und/oder Alkenylrest mit 6 bis 22 Kohlenstoffatomen steht. Vorzugsweise enthalten die erfindungsgemäßen Mischungen als Komponente (b) ungesättigte Aldehyde, die vorzugsweise in trans-Konformation vorliegen und insbesondere 8 bis 12 Kohlenstoffatome aufweisen. Die Mischungen enthalten die Komponenten (a) und (b) vorzugsweise in Mengenverhältnissen von 1:0,00001 bis 1:120, vorzugsweise 1:0,001 bis 1:50, insbesondere 1:0,01 bis 1:10 und besoners bevorzugt 1: 0,1 bis 1:1

Die erfindungsgemäßen Gemiscche zeichnen sich dadurch aus, dass sie sich durch einen intensiven milchig-cremigen Geruch auszeichnen, während die Lactone für sich praktisch geruchsfrei sind und die Aldehyde einen stechenden, fettigen und äüßerst unangenehmen Geruch besitzen.

### Beispiele

### Beispiele 1 und 2, Vergleichsbeispiel V1

Eine kommerziell erhältliche Seifengrundbasis (Soap Base VVF, Henkel Poland) wurde mit jeweils 0,05 Gew.-% einer Mischung (a) aus γ-Decalacton und γ-Undecalacton (Gewichtsverhältnis 1:1) sowie (b) γ-Nonalacton, γ-Decalacton, δ-Decalacton und γ-Undecalacton (Gewichtsverhältnis 1:1:1:1) versetzt und die Geruchssensorik von einem Panel bestehend aus 5 .Intensitätsskala von 1 bis 25. Die Ergebnisse sind in **Tabelle** 1 zusammengefasst. Die Beispiele 1 und 2 sind erfindungsgemäß, Beispiel V1 dient zum Vergleich.

**Tabelle 1**

| Sensorische Beurteilung einer Seifenbasis | | | |
|---|---|---|---|
| **Geruchsnote** | **V1** | **1** | **2** |
| ***Positive Geruchsnoten*** | | | |
| süßlich | 1 | 17 | 18 |
| cremig | 4 | 13 | 12 |
| blumig | 1 | 2 | 2 |
| aromatisch | 2 | 1 | 1 |

| ***Negative Geruchsnoten*** | | | |
|---|---|---|---|
| fettig | 4 | 1 | 1 |
| oxidiert | 11 | 2 | 2 |
| chemisch | 2 | 2 | 2 |
| muffig | 1 | 1 | 1 |

Die Beispiele und das Vergleichsbeispiel zeigen, dass durch den Zusatz schon kleiner Mengen an Lactonen nicht nur die negativen Geruchsnoten der Seifenmischung maskiert werden, sondern stattdessen angenehme Geruchsnoten in den Vordergrund treten

Nachfolgend sind Formulierungsbeispiele für verschiedene Wasch-, Spül- und Reinigungsmittel angegeben, die die erfindungsgemäß zu verwendenden Lactone enthalten. Als Produkt NeoScent AA wird eine Mischung aus γ-Nonalacton, γ-Decalacton, δ-Decalacton und γ-Undecalacton (Gewichtsverhältnis 1:1:1:1) eingesetzt. Alle Mengenangaben verstehen sich als Gew.-%.

**Tabelle 1:**

| Stückseifen | | | |
|---|---|---|---|
| **Zusammensetzung** | **A1** | **A2** | **A3** |
| Sodium C12/14 Olefin Sulfonate | 40,0 | - | - |
| Disodium Lauryl Sulfosuccinate | - | 40,0 | - |
| Lauryl Glucoside | 15,0 | 15,0 | 55,0 |
| Cetylstearyl Alcohol | 12,0 | 12,0 | 12,0 |
| Paraffin Oil (Smp. 54 bis 56 °C) | 8,0 | 8,0 | 8,0 |
| Maze Starch, degradated | 8,0 | 8,0 | 8,0 |
| Palmitic Acid (and) Stearic Acid | 5,5 | 5,5 | 5,5 |
| Glyceryl Stearate | 2,0 | 2,0 | 2,0 |
| Coco Glyceride | 2,0 | 2,0 | 2,0 |
| Polyquaternium-7 | 1,0 | 1,0 | 1,0 |
| Parfümöl | 1,0 | 1,0 | 1,0 |
| Titandioxid | 0,5 | 0,5 | 0,5 |
| NeoScent AA | 0,05 | 0,05 | 0,05 |
| Wasser | ad 100 | | |

**Tabelle 2**

| Syndetseifen | | | |
|---|---|---|---|
| **Zusammensetzung** | **B1** | **B2** | **B3** |
| Lauryl Glucoside | 15,0 | 15,0 | 15,0 |
| Sodium C12/14 Olefin Sulfonate | 40,0 | 40,0 | - |
| Sodium Lauryl Sulfate | - | - | 40,0 |
| Paraffin Oil (Smp. 54 °C) | 8,0 | 8,0 | 8,0 |
| Tallow Fatty Alcohol | 7,0 | 7,0 | 7,0 |
| Maisstärke | 17,0 | - | 17,0 |
| Dextrose | - | 17,0 | - |
| Coco Fatty Acids | 10,0 | 10,0 | 10,0 |
| Titandioxid | 1,0 | 1,0 | 1,0 |
| Parfümöl | 1,0 | 1,0 | 1,0 |
| NeoScent AA | 0,05 | 0,05 | 0,05 |
| Wasser | ad 100 | | |

**Tabelle 3**

| WC-Gel Toilettensteine | | | | | | |
|---|---|---|---|---|---|---|
| **Zusammensetzung** | **C1** | **C2** | **C3** | **C4** | **C5** | **C6** |
| Lauryl Glucoside | 5,0 | 1,5 | 1,5 | 1,5 | 5,0 | 1,5 |
| Decyl Glucoside | - | 3,5 | 3,5 | 3,5 | - | 3,5 |
| Sodium Lauryl Sulfate | 9,0 | 6,0 | 3,0 | - | 9,0 | 6,0 |
| Sodium Laureth Sulfate | - | 3,0 | - | - | - | 3,0 |
| Cocamidopropyl Betaine | - | - | 6,0 | 9,0 | - | - |
| Ethanol | 3,5 | 3,5 | 3,5 | 3,5 | 3,5 | 3,5 |
| Citric Acid Mono Hydrate | 3,6 | 3,6 | 3,6 | 3,6 | 3,6 | 3,6 |
| Sodium Hydroxide | 0,5 | 0,5 | 0,5 | 0,5 | 0,5 | 0,5 |
| Laurylamin+1EO | 4,5 | 3,0 | 5,0 | 2,5 | - | - |
| Hexandiol-1,6 | - | 0,5 | - | | 0,5 | - |
| Parfümöl | 0,5 | 0,5 | 0,5 | 0,5 | 0,5 | 0,5 |
| NeoScent AA | 0,05 | 0,05 | 0,05 | 0,05 | 0,05 | 0,05 |
| Wasser | ad 100 | | | | | |

**Tabelle 4**

| Bleichlaugen | | | | | |
|---|---|---|---|---|---|
| **Zusammensetzung** | **D1** | **D2** | **D3** | **D4** | **D5** |
| Natriumhypochlorit | 4,0 | 4,0 | 4,0 | 4,0 | 4,0 |
| Natriumhydroxid | 0,9 | 0,9 | 0,9 | 0,9 | 0,9 |
| Sodium Laureth-2 Sulfate | - | 2,0 | 2,0 | - | 2,0 |
| Lauryldimethylaminoxid | - | 2,0 | - | - | 2,0 |
| Coco Glucosides | - | - | 0,5 | - | - |
| Laureth-2 | - | - | 1,0 | - | - |
| Natriumsilicat | 0,95 | 0,95 | 0,95 | 0,95 | 0,95 |
| Natriumcarbonat | - | - | - | - | - |
| Aminoxidphosphonsäure | 0,1 | 0,1 | 0,1 | 01 | 0,1 |
| Polyacrylat | 1,0 | 1,0 | 1,0 | 1,0 | 1,0 |
| Parfümöl | 0,5 | 0,5 | 0,5 | 0,5 | 0,5 |
| NeoScent AA | 0,05 | 0,05 | 0,05 | 0,05 | 0,05 |
| Wasser | ad 100 | | | | |

**Tabelle 5**

| Pulverwaschmittel | | | | | | |
|---|---|---|---|---|---|---|
| **Zusammensetzung** | **E1** | **E2** | **E3** | **E4** | **E5** | **E6** |
| Sodium Laureth-1 Sulfate | 12,0 | 8,0 | 8,0 | 4,0 | 4,0 | - |
| Dodecylbenzol Sulfonate | - | - | 4,0 | 8,0 | 4,0 | 8,0 |
| Sodium Lauryl Sulfate | - | 4,0 | - | - | 4,0 | 4,0 |
| Coco Fatty Acid, Sodium Salt | 1,0 | 1,0 | 1,0 | 1,0 | 1,0 | 1,0 |
| Coco Glucosides | - | - | - | 7,0 | - | - |
| Laureth-5 | 7,0 | 7,0 | 7,0 | - | 7,0 | 7,0 |
| Zeolith A | 24,0 | 24,0 | 24,0 | 24,0 | 24,0 | 24,0 |
| Natriumsilicat, amorph (Modul 1:2) | 3,0 | 3,0 | 3,0 | 3,0 | 3,0 | 3,0 |
| Natriumcarbonat | 12,0 | 12,0 | 12,0 | 12,0 | 12,0 | 12,0 |
| Polymeres Polycarboxylat | 5,5 | 5,5 | 5,5 | 5,5 | 5,5 | 5,5 |
| Perboratmonohydrat | 16,0 | 16,0 | 16,0 | 16,0 | 16,0 | 16,0 |
| Tetracetylethylendiamin | 5,5 | 5,5 | 5,5 | 5,5 | 5,5 | 5,5 |
| Carboxymethylcellulose | 0,3 | 0,3 | 0,3 | 0,3 | 0,3 | 0,3 |
| Optischer Aufheller | 0,2 | 0,2 | 0,2 | 0,2 | 0,2 | 0,2 |
| Enzymgranulat (Protease) | 1,0 | 1,0 | 1,0 | 1,0 | 1,0 | 1,0 |
| Parfümöl | 0,5 | 0,5 | 0,5 | 0,5 | 0,5 | 0,5 |
| NeoScent AA | 0,05 | 0,05 | 0,05 | 0,05 | 0,05 | 0,,05 |
| Wasser | ad 100 | | | | | |

**Tabelle 6**

| Handgeschirrspülmittel | | | | |
|---|---|---|---|---|
| **Zusammensetzung** | **F1** | **F2** | **F3** | **F4** |
| Sodium Laureth-1 Sulfate | 15 | 15 | 15 | 15 |
| Sodium Laureth-2 Sulfate | 15 | - | - | - |
| Sodium Lauryl Sulfate | - | 15 | - | - |
| Coco Glucosides | - | - | 15 | - |
| Sodium Octyl Sulfosuccinate | - | - | | 15 |
| Cocamidopropyl Betaine | 1 | 3 | 3 | 3 |
| Parfümöl | 0,5 | 0,5 | 0,5 | 0,5 |
| NeoScent AA | 0,05 | 0,05 | 0,05 | 0,05 |
| Wasser | ad 100 | | | |

**Tabelle 7**

| Reinigungsmittel | | | | | | |
|---|---|---|---|---|---|---|
| **Zusammensetzung** | **G1** | **G2** | **G3** | **G4** | **G5** | **G6** |
| C_{12/16}-Alkylsulfat-Na-Salz | 5,0 | 5,0 | 4,0 | 0 | 5,0 | 0 |
| C_{16/18}-Talgfettalkohol+25EO | 3,0 | 3,0 | 4,0 | 0 | 0 | 5,0 |
| C_{12/14}-Kokosalkyloligoglucasid | 1,0 | 2,0 | 2,0 | 5,0 | 0 | 0 |
| Natriummetasilicat | 15,0 | 15,0 | 15,0 | 15,0 | 15,0 | 15,0 |
| Natriumcarbonat | 26,0 | 24,0 | 24,0 | 30,0 | 30,0 | 30,0 |
| Sulfoniertes Styrol/MSA Copolymer, Natriumsalz | - | 2,0 | 2,0 | - | - | - |
| Limonen | 0,1 | 0.1 | 0,1 | 0,1 | 0,1 | 0,1 |
| NeoScent AA | 0,05 | 0,05 | 0,05 | 0,05 | 0,05 | 0,05 |
| Natriumtripolyphosphat | ad 100 | | | | | |

**Tabelle 8**

| Kosmetische Cremes | | | | | |
|---|---|---|---|---|---|
| **Zusammensetzung** | **H1** | **H2** | **H3** | **H4** | **H5** |
| Glyceryl Sterate (and) Ceteareth 12/20 (and) Cetearyl Alcohol (and) Cetyl Palmitate | 5,0 | 5,0 | 4,0 | - | - |
| Ceteareth-12 | - | - | 1,0 | - | - |
| Polyglyceryl-3 Isostearate | - | - | - | 4,0 | - |
| Polyglyceryl-2 Dipolyhydroxystearate | - | - | - | - | 4,0 |
| Glyceryl Oleate | - | - | - | 2,0 | - |
| PEG-7 Glyceryl Cocoate | - | - | - | - | 2,0 |
| Dicaprylyl Ether | - | - | - | 5,0 | 6,0 |
| Hexyldecanol (and) Hexyldecyl Laurate | - | - | 3,0 | 10,0 | 9,0 |
| Cetearyl Isononanoate | 3,0 | 3,0 | - | - | - |
| Decyl Oleate | 3,0 | 3,0 | - | - | - |
| Coco Caprylate Caprate | - | - | 3,0 | 5,0 | 5,0 |
| Bienenwachs | - | - | - | 7,0 | 5,0 |
| Hydrolyzed Elastin | 2,0 | - | - | - | - |
| Hydrolyzed Collagen | - | 2,0 | - | - | - |
| Hydrolyzed Wheat Gluten | - | - | 0,5 | - | - |
| Sodium Cocoyl Hydrolyzed Wheat Protein | - | - | - | 0,5 | 0,5 |
| Glycol Distearate (and) Laureth-4 (and) Cocamidopropyl Betaine | 5,0 | 5,0 | 4,0 | - | - |
| Glycerin (86 Gew.-%ig) | - | - | 1,0 | - | - |
| Parfümöl | 0,5 | 0,5 | 0,5 | 0,5 | 0,5 |
| NeoScent AA | 0,05 | 0,05 | 0,05 | 0,05 | 0,05 |
| Wasser | ad 100 | | | | |

## Patentansprüche

1. Verwendung einer Mischung enthaltend
(a) γ-Decalacton und
(b) γ-Undecalacton
zur Maskierung von geruchlichen Fehlnoten in oleochemischen Zubereitungen.

2. Verwendung nach Anspruch 1, **dadurch gekennzeichnet, dass** man Mischungen einsetzt, die weitere Lactone enthalten, die ausgewählt sind aus der Gruppe, die gebildet wird von γ-Hexalacton, γ-Heptalacton, γ-Octalacton, γ-Nonalacton, γ-Dodecalacton, δ-Hexalacton, δ-Heptalacton, δ-Octalacton, δ-Nonalacton, δ-Decalacton, δ-Undecalacton und δ-Dodecalacton sowie deren Gemischen.

3. Verwendung nach Anspruch 2, **dadurch gekennzeichnet, dass** man Mischungen einsetzt, die weiterhin γ-Nonalacton und/oder δ-Decalacton enthalten.

4. Verwendung nach mindestens einem der Ansprüche 1 bis 3, **dadurch gekennzeichnet, dass** man die Lactone zur Maskierung von fettigen oder ranzigen Geruchsnoten einsetzt.

5. Verwendung nach mindestens einem der Ansprüche 1 bis 4, **dadurch gekennzeichnet, dass** man die Lactone zur Maskierung von Geruchsnoten einsetzt, die durch freie Fettsäuren, Aldehyde sowie Oxidationsprodukte von Fetten und Ölen hervorgerufen werden.

6. Verwendung nach mindestens einem der Ansprüche 1 bis 5, **dadurch gekennzeichnet, dass** man die Lactone in Seifenbasen einsetzt.

7. Verwendung nach mindestens einem der Ansprüche 1 bis 5, **dadurch gekennzeichnet, dass** man die Lactone in Wasch-, Spül- und Reinigungsmitteln einsetzt.

8. Verwendung nach mindestens einem der Ansprüche 1 bis 5, **dadurch gekennzeichnet, dass** man die Lactone in kosmetischen Zubereitungen einsetzt.

9. Verwendung nach mindestens einem der Ansprüche 1 bis 8, **dadurch gekennzeichnet, dass** man die Lactone in Mengen von 0,01 bis 1 Gew.-% - bezogen auf die Zubereitungen - einsetzt.

10. Mischungen, enthaltend
(a) γ-Decalacton,
(b) γ-Undecalacton und
(c) Aldehyden der Formel (II)
R²CHO (II)
in der R² für einen linearen oder verzweigten, gesättigten oder ungesättigten Alkyl- und/oder Alkenylrest mit 6 bis 22 Kohlenstoffatomen steht.

11. Mischungen nach Anspruch 10, **dadurch gekennzeichnet, dass** es sich bei der Komponente (b) um ungesättigte Aldehyde handelt, die in trans-Konformation vorliegen.

12. Mischungen nach den Ansorüchen 10 und/oder 11. **dadurch gekennzeichnet, dass** sie die Komponenten (a) und (b) in Mengenverhältnissen von 1:0,00001 bis 1:120 einsetzt.

13. Seifenbasis, umfassend eine Mischung enthaltend γ-Decalacton und γ-Undecalacton.

## Claims

1. Use of a mixture comprising
(a) γ-decalactone and
(b) γ-undecalactone
for masking odoriferous incorrect notes in oleochemical preparations.

2. Use according to Claim 1, **characterized in that** mixtures are applied further comprising additional lactones selected from the group consisting of γ-hexalactone, γ-heptalactone, γ-octalactone, γ-nonalactone, γ-dodecalactone, δ-hexalactone, δ-heptalactone, δ-octalactone, δ-nonalactone, δ-decalactone, δ-undecalactone, δ-dodecalactone and their mixtures.

3. Use according to Claim 2, **characterized in that** mixtures are applied further comprising γ-nonalactone and/or δ-decalactone.

4. Use according to any of Claims 1 to 3, **characterized in that** said mixtures are applied for masking greasy or rancid odour notes.

5. Use according to any of Claims 1 to 4, **characterized in that** the lactones are used for masking odour notes which are caused by free fatty acids, aldehydes, and oxidation products of fats and oils.

6. Use according to any of Claims 1 to 5, **characterized in that** the lactones are used in soap bases.

7. Use according to any of Claims 1 to 5, **characterized in that** the lactones are used in laundry detergents, dishwashing detergents and cleaners.

8. Use according to any of Claims 1 to 5, **characterized in that** the lactones are used in cosmetic preparations.

9. Use according to any of Claims 1 to 6, **characterized in that** the lactones are used in amounts of from 0.01 to 1% by weight - based on the preparations.

10. Mixtures comprising
(a) γ-decalactone,
(b) γ-undecalactone, and
(c) aldehydes of formula (II)
R²CHO (II)
In which R² stands for a linear or branched, saturated or unsaturated alkyl and/or alkenyl residue having 6 to 22 carbon atoms.

11. Mixtures according to Claim 10, **characterized in that** said component (b) represents unsaturated aldehydes showing a trans-configuration.

12. Mixtures according to Claim 10 and/or 11, **characterized in that** said components (a) and (b) are applied in ratios by weight of from 1:0.00001 to 1:120.

13. Soap base, comprising a mixture comprising γ-decalactone and γ-undecalactone.

## Revendications

1. Utilisation d'un mélange, contenant
(a) de la γ-décalactone et
(b) de la γ-undécalactone
pour le masquage de mauvaises notes odorantes dans des préparations oléochimiques.

2. Utilisation selon la revendication 1, **caractérisée en ce qu'**on utilise des mélanges qui contiennent d'autres lactones choisies dans le groupe formé par la γ-hexalactone, la γ-heptalactone, la γ-octalactone, la γ-nonalactone, la γ-dodécalactone, la δ-hexalactone, la δ-heptalactone, la δ-octalactone, la δ-nonalactone, la δ-décalactone, la δ-undécalactone et la δ-dodécalactone ainsi que leurs mélanges.

3. Utilisation selon la revendication 2, **caractérisée en ce qu'**on utilise des mélanges qui contiennent en outre de la γ-nonalactone et/ou de la δ-décalactone.

4. Utilisation selon au moins l'une quelconque des revendications 1 à 3, **caractérisée en ce qu'**on utilise les lactones pour le masquage de notes odorantes grasses ou rances.

5. Utilisation selon au moins l'une quelconque des revendications 1 à 4, **caractérisée en ce qu'**on utilise les lactones pour le masquage de notes odorantes qui sont provoquées par des acides gras libres, des aldéhydes ainsi que par des produits d'oxydation de graisses et d'huiles.

6. Utilisation selon au moins l'une quelconque des revendications 1 à 5, **caractérisée en ce qu'**on utilise les lactones dans des bases de savon.

7. Utilisation selon au moins l'une quelconque des revendications 1 à 5, **caractérisée en ce qu'**on utilise les lactones dans des agents de lavage, de rinçage et de nettoyage.

8. Utilisation selon au moins l'une quelconque des revendications 1 à 5, **caractérisée en ce qu'**on utilise les lactones dans des préparations cosmétiques.

9. Utilisation selon au moins l'une quelconque des revendications 1 à 8, **caractérisée en ce qu'**on utilise les lactones en des quantités de 0,01 à 1% en poids par rapport aux préparations.

10. Mélanges, contenant
(a) de la γ-décalactone,
(b) de la γ-undécalactone et
(c) des aldéhydes de formule (II)
R²CHO (II)
dans laquelle R² représente un radical alkyle et/ou alcényle linéaire ou ramifié, saturé ou insaturé, comprenant 6 à 22 atomes de carbone.

11. Mélanges selon la revendication 10, **caractérisés en ce qu'**il s'agit, pour le composant (b), d'aldéhydes insaturés qui se trouvent dans une conformation trans.

12. Mélanges selon les revendications 10 et/ou 11, **caractérisés en ce qu'**ils contiennent les composants (a) et (b) en des rapports quantitatifs de 1:0,00001 à 1:120.

13. Base de savon, comprenant un mélange contenant de la γ-décalactone et de la γ-undécalactone.
